# EUROPEAN PATENT SPECIFICATION

(11) **EP 2 296 758 B1**
(45) Date of publication and mention of the grant of the patent: **23.05.2018**
(21) Application number: 09793905.2
(22) Date of filing: 17.06.2009
(51) Int. Cl.: A61Q 5/12, A61Q 5/04, A61K 8/898, A61K 8/891, C08L 83/08, C08L 83/04, A61Q 5/02, C08G 77/26

(54) **HAIR CONDITIONING COMPOSITION COMPRISING THREE KINDS OF SILICONES**
HAARSPÜLUNG MIT DREI SILIKONPOLYMEREN
COMPOSITION APRÈS-SHAMPOOING COMPRENANT TROIS SILICONES

(30) Priority: 10.07.2008 EP 08160061
(43) Date of publication of application: 23.03.2011
(73) Proprietor: Unilever PLC, London, Greater London EC4P 4BQ (GB); Unilever N.V., 3013 AL Rotterdam (NL)
(72) Inventor: RICHARDS, Claire, Louise, Wirral Merseyside CH63 3JW (GB)
(74) Representative: Chisem, Janet
(86) International application number: PCT/EP2009/057538
(87) International publication number: WO 2010/003793

(56) References cited:
- EP-A- 1 652 555
- WO-A-2004/030646
- US-A- 5 393 521
- US-A- 5 807 545
- US-A- 6 048 519
- US-A1- 2003 082 128

## Description

The present invention relates to an improved hair composition.

Despite the prior art there remains the need for compositions which are able to prevent hair from frizzing post wash and dry.

Accordingly, and in a first aspect, the present invention provides an aqueous composition comprising 0.01 to 5% wt. of an alkyl-modified silicone, which comprises at least one pendant alkyl group having a hydrocarbyl chain length of C₆ or greater extending from at least one of the silicon atoms forming the polymer backbone, the composition additionally comprising a further silicone component comprising (i) from 50 to 95% by weight of the further silicone component, a second silicone which has a viscosity of at least 100,000 mm2/sec at 25°C, and (ii) from 5 to 50% by weight of the further silicone component, a functionalised silicone which is an amino-functionalised silicone, wherein the alkyl modified silicone is characterised by the general formula (I) :

**(CH₃)₃Si-o-[Si(CH₃)(R)O]ₘ-[Si(CH₃)₂O]ₙ-Si(CH₃)₃** **(I)**

in which m has a value of 1 to 450, n has a value of 1 to 3000 and R is a monovalent alkyl radical of from 8 to 60 carbon atoms, and wherein the silicone component has an average D3,2 particle size in the range from 0.004 to 100µm, as measured by laser light scattering using an instrument such as a Malvern Mastersizer™.

The physical form of alkyl modified silicones under ambient conditions generally varies from wax to fluid depending on molecular parameters such as the chain length of the alkyl group, number of alkyl groups (other than methyl) in the molecule and silicone backbone molecular weight.

The term "ambient conditions" as used herein refers to surrounding conditions at one atmosphere of pressure, 50% relative humidity, and 25°C.
Preferred alkyl modified silicones for use in the invention are fluids under ambient conditions.
Preferred alkyl modified silicones for use in the invention have a number average molecular weight (Mₙ) ranging from 10,000 to 450,000, more preferably from 60,000 to 110,000 dalton. The alkyl modified silicones for use in the hair care compositions of the invention are chemically characterised by the general formula (I):

(CH₃)₃Si-O-[Si(CH₃)(R)O]ₘ-[Si(CH₃)₂O]ₙ-Si(CH₃)₃ (I)

in which m has a value of 1 to 450, n has a value of 1 to 3000 and R is a monovalent alkyl radical of from 8 to 60 carbon atoms.
In general formula (I), the -[Si(CH₃)₂O]- units are typically randomly interspersed with the -[Si(CH₃)(R)O]- units. m and n are typically average values due to the nature of the polymerisation process.
Preferred materials of general formula (I) for use in the invention have an m value ranging from 40 to 100, more preferably from 50 to 80.

Preferred materials of general formula (I) for use in the invention have an n value ranging from 500 to 1400, more preferably from 700 to 1200.

Preferred materials of general formula (I) for use in the invention have a ratio of m:n ranging from 15:85 to 1:99, more preferably from 10:90 to 5:95.

In preferred materials of general formula (I) for use in the invention, R is a linear alkyl radical having from 8 to 22, more preferably from 8 to 14, most preferably from 10 to 12 carbon atoms.

Preferably, the alkyl-modified silicones of the invention are emulsified.

Methods for the preparation of alkyl modified silicones suitable for use in the invention are known in the art and described for example in EP 495 596 and WO91/09586.

Alkyl modified silicones suitable for use in the invention are also commercially available from suppliers of silicones such as Momentive Performance Materials, Inc. (of Wilton, Connecticut, USA) and Dow Corning Corporation (of Midland, Michigan, USA).

Emulsified particles of alkyl modified silicone may typically have a Sauter mean particle diameter (D_{3,2}) in the composition of the invention ranging from 0.1 to 10, preferably from 1 to 4 micrometers.

A suitable method for measuring the Sauter mean droplet diameter (D_{3,2}) is by laser light scattering using an instrument such as a Malvern Mastersizer.

Mixtures of any of the above described alkyl modified silicones may also be used.

Alkyl modified silicones for use in compositions of the invention are available as pre-formed silicone emulsions from suppliers of silicones such as those mentioned above. The use of such pre-formed silicone emulsions is preferred for ease of processing and control of silicone particle size. Such pre-formed silicone emulsions will typically additionally comprise a suitable emulsifier, such as an anionic or non-ionic surfactant, and may be prepared by a chemical emulsification process such as emulsion polymerisation, or by mechanical emulsification using a high shear mixer.

The total amount of alkyl modified silicone in hair care compositions of the invention generally ranges from 0.01 to 5%, preferably from 0.05 to 2%, more preferably from 0.1 to 1.5% by total weight alkyl modified silicone based on the total weight of the composition.

Preferably, the composition comprises from 0.1 to 20% wt. further silicone component.

The further silicone component has an average particle size in the range from 0.004 to 100 µm, more preferably from 0.004 to 20µm and most preferably from 0.004 to 10 µm.
Preferably, the further silicone component is in the form of a mechanical emulsion.
A suitable further silicone component is commercially available from Dow Corning as DC 7134.
Preferably, the second silicone is a gum and has a viscosity of at least 500,000 mm2/sec at 25°C. Preferably, the second silicone has a molecular weight of at least 200,000 Daltons.
Preferably, the second silicone is a polydimethylsiloxane.
Preferably, the functionalised silicone has a viscosity of less than 500,000 mm2/sec at 25°C. Preferably, the functionalised silicone has a molecular weight of less than 200,000 Daltons. The functionalised silicone is an amino-functionalised silicone. Preferably, the amino-functionalised silicone has a mole percent amino functionality in the range from 0.3 to 8, preferably from 0.5 to 4.
Preferably, the weight ratio of the second silicone to the functionalised silicone in the silicone component is in the range from 15:1 to 1:1, preferably from 5:1 to 1:5.

The composition according to the invention may be any hair care composition for example a shampoo, conditioner, mask or such composition used as part of the hair cleansing regime. Preferably though the composition is a conditioning composition or a conditioning shampoo. More preferably, it is a dedicated conditioning composition for the treatment of hair (typically after shampooing) and subsequent rinsing.

Such a conditioner will comprise one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, e.g. chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as DEHYQUART, ex Henkel.

In conditioners of the invention, the level of cationic surfactant is preferably from 0.01 to 10%, more preferably 0.05 to 5%, most preferably 0.1 to 2% by weight of the composition.

Conditioners of the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol material in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

Silicone is a particularly preferred ingredient in hair treatment compositions of the invention. In particular, hair shampoos and conditioners of the invention will preferably also comprise emulsified particles of silicone, for enhancing conditioning performance. The silicone is insoluble in the aqueous matrix of the composition and so is present in an emulsified form, with the silicone present as dispersed particles.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188. These materials can impart body, volume and stylability to hair, as well as good wet and dry conditioning.

The viscosity of the emulsified silicone itself (not the emulsion or the final hair conditioning composition) is typically at least 10,000 cst. In general we have found that conditioning performance increases with increased viscosity. Accordingly, the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in hair shampoos and conditioners of the invention will typically have an average silicone particle size in the composition of less than 30, preferably less than 20, more preferably less than 10 microns. We have found that reducing the particle size generally improves conditioning performance. Most preferably the average silicone particle size of the emulsified silicone in the composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in shampoos and conditioners of the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   HO-[Si(CH₃)₂-O-]ₓ-[Si(OH)(CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   R'ₐG₃₋ₐ-Si(OSiG₂)ₙ-(OSiG_{b}R'_{2-b})ₘ-O-SiG₃₋ₐ-R'ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R' is a monovalent radical of formula -C_{q}H_{2q}L in which q is a number from 2 to 8 and L is an aminofunctional group selected from the following:
      -NR"-CH₂-CH₂-N(R")₂
      -N(R")₂
      -N⁺(R")₃A⁻
      -N⁺H(R")₂A⁻
      -N⁺H₂(R")A⁻
      -N(R")-CH₂-CH₂-N⁺H₂(R")A⁻
      in which R" is selected from H, phenyl, benzyl, or a saturated monovalent hydrocarbon radical, e.g. C₁₋₂₀ alkyl, and;
   A is a halide ion, e.g. chloride or bromide.

   Suitable amino functional silicones corresponding to the above formula include those polysiloxanes termed "trimethylsilylamodimethicone" as depicted below, and which are sufficiently water insoluble so as to be useful in compositions of the invention:

   Si(CH₃)₃-O-[Si(CH₃)₂-O-]ₓ-[Si(CH₃)(R-NH- CH₂CH₂NH₂)-O-]_{y}-Si(CH₃)₃

   wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³)N⁺CH₂CH(OH)CH₂O(CH₂)₃[Si(R⁴)(R⁵)-O-]ₙ-Si(R⁶)(R⁷)- (CH₂)₃-O-CH₂CH(OH)CH₂N⁺(R⁸)(R⁹)(R¹⁰)} (X⁻)₂

   wherein R¹ and R¹⁰ may be the same or different and may be independently selected from H, saturated or unsaturated long or short chain alk(en)yl, branched chain alk(en)yl and C₅-C₈ cyclic ring systems;
   R² thru' R⁹ may be the same or different and may be independently selected from H, straight or branched chain lower alk(en)yl, and C₅-C₈ cyclic ring systems;
   n is a number within the range of about 60 to about 120, preferably about 80, and
   X⁻ is preferably acetate, but may instead be for example halide, organic carboxylate, organic sulphonate or the like. Suitable quaternary silicone polymers of this class are described in EP-A-0 530 974.

Amino functional silicones suitable for use in shampoos and conditioners of the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functional silicone is not particularly critical and can suitably range from about 100 to about 500,000 cst.

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).

Also suitable are emulsions of amino functional silicone oils with non ionic and/or cationic surfactant.
Suitably such pre-formed emulsions will have an average amino functional silicone particle size in the shampoo composition of less than 30, preferably less than 20, more preferably less than 10 microns. Again, we have found that reducing the particle size generally improves conditioning performance. Most preferably the average amino functional silicone particle size in the composition is less than 2 microns, ideally it ranges from 0.01 to 1 micron. Silicone emulsions having an average silicone particle size of ≤ 0.15 microns are generally termed microemulsions.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

An example of a quaternary silicone polymer useful in the present invention is the material K3474, ex Goldschmidt.

The total amount of silicone incorporated into compositions of the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy.

We have found that a total amount of silicone of from 0.3 to 5%, preferably 0.5 to 3%, by weight of the total composition is a suitable level.

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   - free fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

The invention is further illustrated by way of the following non-limitative Examples, in which all percentages quoted are by weight based on total weight unless otherwise stated.

In a second aspect there is provided a method for preventing frizzing of hair by applying to the hair a composition according to the first aspect of the invention.

### EXAMPLE 1

A composition according to an embodiment of the invention. It is made by standard processes.

| **Chemical name** | | **% active** | **% wt** |
|---|---|---|---|
| Water | Water | 100.00 | To 100 |
| Ethylendiaminetetra acetic acid | Preservative | 100.00 | 0.10 |
| Lactic Acid | Purac 88S | 88.00 | 0.33 |
| Preservative | Glydant | 100.00 | 0.14 |
| Stearyl Alcohol | Lanette S3 | 100.00 | 5.00 |
| Behentrimonium chloride | BTLF | 70.00 | 0.875 |
| Stearamidopropyl dimethylamine | Lexamine S13 | 100.00 | 1.250 |
| Potassium Chloride | Potassium Chloride | 100.00 | 0.100 |
| Alkylmethylsiloxane | HUJ 7419* | 60.00 | 3.00 |
| Aminosilicone/ dimethicone | DC5-7134** | 70.00 | 3.00 |

| | | | |
|---|---|---|---|
| * HUJ-7419 silicone emulsion (INCI name is alkylmethyl silicone) ex. Momentive. ** 7134 is DC 5-7 134 9:1 600K/8566 CTAC 70% (INCI name is Dimethicone/amino silicone emulsion) ex. Dow Corning. | | | |

### EXAMPLE 2

A comparison between a composition according to an embodiment of the invention and two which are not.

Panellists were asked to compare switches washed and dried with the test compositions with standards representing high and low frizz.

| **Composition** | **Frizz value (error)** |
|---|---|
| 3% HUJ | 6.5 (1.0) |
| 3% HUJ, 3% DC 7134 | 3.1 (1.1) |
| 3% DC 7134 | 6.9 (0.7) |

The results in the table show that each of the alkyl-modified silicone and the amino silicone provide a certain level of frizz reduction while the mixture of the alkyl modified silicone and the amino silicone surprisingly provide a significant reduction in frizz. A low value represents a low frizz.

## Claims

1. Aqueous composition comprising 0.01 to 5% wt. of an alkyl-modified silicon which comprises at least one pendant alkyl group having a hydrocarbyl chain length of C₆ or greater extending from at least one of the silicon atoms forming the polymer backbone, the composition additionally comprising a further silicon component comprising (i) from 50 to 95% by weight of the further silicone component, a second silicone which has a viscosity of at least 100,000 mm²/sec at 25°C, and (ii) from 5 to 50% by weight of the further silicone component, a functionalised silicone which is an amino-functionalised silicone, wherein the alkyl modified silicone is **characterised by** the general formula (I):
**(CH₃)₃Si-o-[Si(CH₃)(R)O]ₘ-[Si(CH₃)₂O]ₙ-Si(CH₃)₃** **(I)**
in which m has a value of 1 to 450, n has a value of 1 to 3000 and R is a monovalent alkyl radical of from 8 to 60 carbon atoms, and wherein the silicone component has an average D3,2 particle size in the range from 0.004 to 100µm, as measured by laser light scattering using an instrument such as a Malvern Mastersizer™.

2. Aqueous composition according to claim 1 comprising from 0.1 to 20% wt. further silicone component.

3. A composition according to any preceding claim in which the second silicone is a gum and has a viscosity of at least 500,000 mm²/sec at 25°C.

4. A composition according to any preceding claim in which the functionalised silicone has a viscosity of less than 500,000 mm²/sec at 25°C.

5. A composition according to any preceding claim in which the second silicone has a molecular weight of at least 200,000 Daltons.

6. A composition according to any preceding claim in which the functionalised silicone has a molecular weight of less than 200,000 Daltons.

7. A composition according to any preceding claim, in which the second silicone is a polydimethylsiloxane.

8. A composition according to any preceding claim which is a hair conditioning composition.

9. A composition according to any preceding claim which is a conditioning shampoo.

10. Method for preventing or reducing hair frizz by applying to the hair a composition according to any preceding claim and then shampooing and rinsing.

## Patentansprüche

1. Wässrige Zusammensetzung, umfassend 0,01 bis 5 Gew.-% eines Alkylmodifizierten Silicons, welches mindestens eine anhängende Alkylgruppe mit einer Hydrocarbylkettenlänge von C₆ oder größer, die sich von mindestens einem der das Polymergrundgerüst bildenden Siliconatome erstreckt, umfasst, wobei die Zusammensetzung zusätzlich eine weitere Siliconkomponente umfasst, umfassend (i) von 50 bis 95 Gewichts-% der weiteren Siliconkomponente eines zweiten Silicons, das eine Viskosität von mindestens 100.000 mm²/s bei 25°C aufweist, und (ii) von 5 bis 50 Gewichts-% der weiteren Siliconkomponente eines funktionalisierten Silicons, das ein Amino-funktionalisiertes Silicon darstellt, wobei das Alkyl-modifizierte Silicon durch die allgemeine Formel (I)
(CH₃)₃Si-O-[Si(CH₃)(R)O]ₘ-[Si(CH₃)₂O]ₙ-Si(CH₃)₃ (I)
charakterisiert ist, worin
m einen Wert von 1 bis 450 hat,
n einen Wert von 1 bis 3000 hat und
R ein einwertiger Alkylrest mit 8 bis 60 Kohlenstoffatomen ist und
worin die Siliconkomponente eine durchschnittliche Teilchengröße D3,2 in dem Bereich von 0,004 bis 100 µm, gemessen mit Laserlichtstreuung unter Verwendung eines Geräts, wie eines Malvern Mastersizers^{Wz}, aufweist.

2. Wässrige Zusammensetzung nach Anspruch 1, umfassend von 0,1 bis 20 Gew.-% weitere Siliconkomponente.

3. Zusammensetzung nach irgendeinem vorhergehenden Anspruch, in welcher das zweite Silicon ein Gummi ist und eine Viskosität von mindestens 500.000 mm²/s bei 25°C aufweist.

4. Zusammensetzung nach irgendeinem vorhergehenden Anspruch, in welcher das funktionalisierte Silicon eine Viskosität von weniger als 500.000 mm²/s bei 25°C aufweist.

5. Zusammensetzung nach irgendeinem vorhergehenden Anspruch, in welcher das zweite Silicon ein Molekulargewicht von mindestens 200.000 Dalton aufweist.

6. Zusammensetzung nach irgendeinem vorhergehenden Anspruch, in welcher das funktionalisierte Silicon ein Molekulargewicht von weniger als 200.000 Dalton aufweist.

7. Zusammensetzung nach irgendeinem vorhergehenden Anspruch, in welcher das zweite Silicon ein Polydimethylsiloxan darstellt.

8. Zusammensetzung nach irgendeinem vorhergehenden Anspruch, welche eine Haarkonditionierzusammensetzung darstellt.

9. Zusammensetzung nach irgendeinem vorhergehenden Anspruch, welche ein Konditioniershampoo darstellt.

10. Verfahren zum Verhindern oder Reduzieren von Haarkräuseln durch Auftragen einer Zusammensetzung nach irgendeinem vorhergehenden Anspruch auf das Haar und folgendes Shampoonieren und Ausspülen.

## Revendications

1. Composition aqueuse comprenant de 0,01 à 5 % en poids d'une silicone modifiée par alkyle qui comprend au moins un groupe alkyle pendant ayant une longueur de chaîne hydrocarbyle de C₆ ou supérieure s'étendant à partir d'au moins l'un des atomes de silicium formant le squelette de polymère, la composition comprenant en outre un composant silicone supplémentaire comprenant (i) de 50 à 95 % en poids du composant silicone supplémentaire, une seconde silicone qui a une viscosité d'au moins 100 000 mm²/s à 25 °C, et (ii) de 5 à 50 % en poids du composant silicone supplémentaire, une silicone fonctionnalisée qui est une silicone fonctionnalisée par amino, dans laquelle la silicone modifiée par alkyle est **caractérisée par** la formule générale (I) :
**(CH₃)₃Si-o-[Si(CH₃)(R)O]ₘ-[Si(CH₃)₂O]ₙ-Si(CH₃)₃** **(I)**
dans laquelle m a une valeur de 1 à 450, n a une valeur de 1 à 3 000 et R est un radical alkyle monovalent de 8 à 60 atomes de carbone, et dans laquelle le composant silicone a une taille de particule D3,2 moyenne dans la plage de 0,004 à 100 µm, telle que mesurée par diffusion de la lumière laser à l'aide d'un instrument tel qu'un Malvern Mastersizer™.

2. Composition aqueuse selon la revendication 1 comprenant de 0,1 à 20 % en poids de composant silicone supplémentaire.

3. Composition selon une quelconque revendication précédente dans laquelle la seconde silicone est une gomme et a une viscosité d'au moins 500 000 mm²/s à 25 °C.

4. Composition selon une quelconque revendication précédente dans laquelle la silicone fonctionnalisée a une viscosité inférieure à 500 000 mm²/s à 25 °C.

5. Composition selon une quelconque revendication précédente dans laquelle la seconde silicone a un poids moléculaire d'au moins 200 000 Daltons.

6. Composition selon une quelconque revendication précédente dans laquelle la silicone fonctionnalisée a un poids moléculaire inférieur à 200 000 Daltons.

7. Composition selon une quelconque revendication précédente, dans laquelle la seconde silicone est un polydiméthylsiloxane.

8. Composition selon une quelconque revendication précédente qui est une composition de conditionnement des cheveux.

9. Composition selon une quelconque revendication précédente qui est un shampooing conditionneur.

10. Procédé de prévention ou de réduction du frisottement des cheveux par application sur les cheveux d'une composition selon une quelconque revendication précédente et ensuite réalisation d'un shampooing et d'un rinçage.
